# EUROPEAN PATENT APPLICATION

(11) **EP 0 937 389 A2**
(43) Date of publication of application: **25.08.1999**
(21) Application number: 99102924.0
(22) Date of filing: 13.02.1999
(51) Int. Cl.: A01K 67/027, G01N 33/50

(54) **Scid mouse having a normal immun response to exogenous antigens**

(30) Priority: 24.02.1998 US 28411
(71) Applicant: HEALTH RESEARCH, INC., Buffalo New York 14263 (US)
(72) Inventor: Bankert, Richard B., Eden, NY 14057 (US); Chen, Fang-an, Williamsville, NY 14221 (US); Egilmez, Nejat K., E. Amherst, NY 14051 (US)
(74) Representative: Weber, Dieter, Dr.

(57) **Abstract**

An immunodeficient mammal, e.g. a mouse, having engrafted human lymphocytes which retain the ability to show a normal human immune response. The human lymphocytes in these mammals, e.g. mice, no longer have a xeno-response of the human lymphocytes but do have the ability to respond to exogenous antigens, essentially from the time of engrafting of the human lymphocytes into the mice. This has resulted in an animal model that is able to test the efficacy of human immunotherapeutic protocols. Further, by combining the xenotolerant human lymphocytes (huPBL) with human tumors, human anti-tumor immunity can be monitored. The invention also includes the method of using the mammal to test human immune response.

## Description

### Background of the Invention

This invention relates to immunodeficient mammals, e.g. mice, used for study of immune response and more particularly relates to such mice engrafted with human blood lymphocytes.

If one were able to engraft human lymphocytes into immunodeficient mammals, e.g. mice, and have these cells retain immunological competence, one would have the potential to monitor human lymphocyte responses to immune stimulation and thereby to evaluate vaccines and immunotherapeutic protocols. Further if one were able to co-engraft human tumors and patients' lymphocytes, one would be able to monitor a patients' anti-tumor immunity and to recognize the ability of immunotherapeutic strategies to augment this anti-tumor immunity.

Up to the time of this invention, there were no adequate animal models with which to effectively monitor the immune response of humans.

It has been known from, e.g. "Human Antibody Response in Human Peripheral Blood Leukocyte/Severe Combined Immunodeficient Chimeric Model Is Dependent on B and T Cell Costimulation Via CD40/CD40 Ligand", Chen et al, Journal of Immunology, 1995, that severe combined immunodeficient (SCID) mice could be created which comprise engrafted human lymphocytes (peripheral blood leukocytes, (huPBL)). The engrafted mice did not show normal human immune response to exogenous antigens (antigens, other than mouse antigens), but developed an anemia which seemed to result from production of human anti-mouse erythrocyte antibodies and a graft v host disease. The resulting mice were therefore unsuitable for human immune response studies both because of the unhealthy nature of the mice and because normal human response to non-mouse antigens did not occur.

Human lymphocytes were then treated with a blocking agent selected from Anti-CD-40 monoclonal antibody (mAb) M3 (100µg/mouse), M3-F(ab')₂ (75µg/mouse) or anti-CD40L mAb M91 (100µg/mouse), or the species and isotype matching control mAb 2C3 (100µg/mouse) or chimeric molecule CD-40-Fc (100µg/mouse). The lymphocytes were mixed with the blocking agents two hours before or immediately after engrafting into the immunodeficient (SCID) mice followed by using a half dose of the same blocking agents i.p on day 2 and day 4 after engraftment.

As a result of the above, especially using the antibodies specific for CD40, CD40 ligand (CD40L), and CD40-Fc, response to mouse antigens were blocked. The above document makes no reference to responses by the engrafted lymphocytes to antigens other than mouse antigens. Unfortunately, it was subsequently determined that all other responses by the lymphocytes were also blocked for a period of weeks. These mice were therefore unsuitable for studying human immune response, especially within the first couple of weeks after engraftment.

### Brief Description of the Drawings

Figure 1 shows a comparison of SCID mice engrafted with xenotolerant PBL cells inoculated with dendritic cells against an uninoculated control.
Figure 2 shows a comparison of SCID mice engrafted with xenotolerant and non-xenotolerant PBL's, challenged with prostate specific antigen expressing tumor cells and tested for growth of the tumor cells with prostate specific antigen.

### Brief Description of the Invention

In accordance with the present invention an immunodeficient mammal, e.g. a mouse, having engrafted human lymphocytes, has been developed which has the ability to show a normal human immune response. The human lymphocytes in these mammals, e.g.mice, no longer have a xeno-response of the human lymphocytes but do have the ability to respond to exogenous antigens, essentially from the time of engrafting of the human lymphocytes into the mice. This has resulted in an animal model that is able to test the efficacy of human immunotherapeutic protocols. Further, by combining the xenotolerant human lymphocytes (huPBL) with human tumors, human anti-tumor immunity can be monitored.

More particularly, the invention comprises a severe combined immunodeficient mouse comprising engrafted human peripheral blood lymphocytes, said human peripheral blood lymphocytes being rendered tolerant to mouse tissue antigens prior to said engrafting, said human blood lymphocytes having the ability to respond to non-mouse exogenous antigens from the time of said engrafting.

It has been found that the human blood lymphocytes may be rendered tolerant to mouse tissue antigens prior to engrafting by blocking co-stimulatory molecules on the lymphocytes without further blocking treatment of the lymphocytes after engrafting to obtain such tolerance.

The co-stimulatory blocking may be achieved by numerous methods; provided that, further treatment to obtain such blocking, is not done after engrafting.

The invention further includes a method for monitoring human blood lymphocytic immune response by challenging the mouse of the invention with an antigen normally foreign to both human blood lymphocytes and to mice. The antigen may, for example be found within a vaccine, within a tumor or within a virus.

### Detailed Description of the Invention

Essentially any immune deficient mouse, or for that matter other mammal, can be used in accordance with the present invention. Examples of immunodeficient mammals that can be used include rats, mice, guinea pigs, rabbits, cats, dogs and primates. The mammal, e.g. mouse, must simply be almost completely immune deficient, i.e. must show almost no immune response to antigens to which a normal mammal would respond. The mammals used for purposes of illustration herein, are severe immune deficient (SCID) mice. It is to be understood that any severe immune deficient mammal (except humans for ethical reasons) could be used. SCID mice are readily available from numerous sources.

The human lymphocytes can be readily obtained from human blood by known means, e.g. by leukophoresis of peripheral blood obtained from healthy donors. Red blood cells can be lysed with e.g. 0.83 percent NH₄Cl followed by washing PBL's with prewarmed Hank's solution containing 0.5% bovine serum albumin.

Numerous known blocking agents can be used to render the human lymphocytes tolerant to mouse (or other mammal) antigens prior to engrafting into the mammal. Such blocking agents may be in the public domain or proprietary. The lymphocytes can, for example, be treated with a blocking agent selected from Anti-CD-40 monoclonal antibody (mAb) M3 (100µg/mouse), M3-F(ab')₂ (75µg/mouse) or anti-CD40L mAb M91 (100µg/mouse), or the species and isotype matching control mAb 2C3 (100µg/mouse) or chimeric molecule CD-40-Fc (100µg/mouse). Other methods for rendering the human lymphocytes tolerant to host antigens include blocking the CD28 molecule on human T cells with the human CTLA-4 fusion protein and subsequently exposing the cells to host (e.g. mouse) tissue antigens and antigen presenting cells. This may be accomplished by incubating 3-5 x 10⁷ huPBL with 30 µg of human CTLA-Fc fusion protein and injecting the cell suspension intraperitoneally into the host mammal (mouse). Tolerance to host mammal antigens might also be established by inoculating the huPBL along with an immunosuppressive drug. This method is probably not preferred due to potential reduction in normal response to non-host antigens. Tolerance might also be accomplished by exposure of the lymphocytes to high or low zone doses of antigen prior to innoculation. In all cases, to obtain the test mammal of the invention, lymphocytes are mixed with the blocking agents prior to engrafting into the immunodeficient mammal, e.g. (SCID) mouse without subsequent exposure after engraftment.

The method of monitoring, according to the invention, preferably uses the test mammal of the invention (usually an engrafted SCID mouse) but may use such a mouse subsequently treated by later exposure to blocking agent, e.g. i.p on day 2 and day 4 after engraftment. Such later treated mice are not preferred due to low human immune response for a significant time after engraftment, but might possibly be used from from about one to about three weeks after engraftment.

In more detail, human peripheral blood lymphocytes (either from normal donors or from cancer patients) are rendered tolerant to mouse tissue antigens by blocking co-stimulatory molecules on the surface of lymphocytes just prior to their engraftment into SCID mice. This blocking may be achieved by the addition of the human CD40Fc fusion protein. This fusion protein binds to the CD40 ligand on human T lymphocytes. When the T cells are exposed to mouse tissue antigens (with their CD40 ligand co-stimulatory molecules blocked by the soluble CD40Fc fusion protein) they become specifically non-responsive (i.e., tolerant) to mouse tissue antigens. It has been determined that these xenotolerant huPBL (but not control huPBL) are able subsequently to respond to exogenous antigens (viz. an Epstein Barr Viral antigen). SCID mice engrafted with the xenotolerant human PBL (i.e., xeno.tol. huPBL-SCID mice) and vaccinated with an EBV glycoprotein are able to withstand a subsequent lethal challenge with Epstein Barr Virus. Also xeno.tol huPBL-SCID mice are able to reject a tumor xenograft (of a human lung tumor) whereas conventional huPBL-SCID mice do not reject the tumor xenograft. These results sustain the notion that huPBL injected intraperitoneally into SCID mice and rendered tolerant of the host (SCID mouse) tissue antigens are able to respond to exogenous antigens. Our data establish that this modified huPBL SCID model is able to demonstrate and monitor human anti-tumor immunity.

We also enhance the engraftment of human PBL and human tumor xenografts in the SCID mouse by eliminating the mouse NK cell activity with the administration of anti-NK cell antibodies.

This model is expected to serve as a simple and reliable method for evaluating cancer vaccines and novel immunotherapeutic protocols for cancer therapy (i.e., dendritic cell peptide protocols, recombinant cytokine administration or cytokine gene therapies). It also represents an opportunity to test the efficacy of other vaccines (i.e., for AIDS, hepatitis, etc.).

The following specific examples serve to illustrate but not limit the present invention. It is to be understood that the principles applied to mice are equally applicable to other mammals.

### Method used to induce Xenotolerance in Human Lymphocytes Engrafted into SCID Mice:

Human peripheral blood lymphocytes (huPBL) are mixed with a CD40Fc fusion protein just prior to inoculation into SCID mice. 3 - 5 x 10⁷ huPBL are resuspended with 30 µg of human CD40Fc fusion protein and injected intraperitoneally into each SCID mouse. Mice are immunized or challenged with tumor 7-15 days after the inoculation of either the xenotolerant (CD40Fc treated) or control huPBL.

### Examples Demonstrating that Xenotolerance of huPBL Enhances Functional Immunity:

Three examples (with data) are presented that support the notion that xenotolerance enhances the functional immune capacity of human PBL engrafted in the SCID mouse.

### Example 1. Use of Xenotolerance Model to Evaluate Ability of Tumor Primed Dendritic Cells to Augment huPBL Mediated Anti-Tumor Immunity.

A non-small cell lung tumor was established in culture from tumor biopsy tissue obtained from a lung cancer patient. PBL were obtained by leukophoresis. These PBL were used to engraft SCID mice by inoculating 5 x 10⁷ cells intraperitoneally with or without establishing xenotolerance by the addition of CD40Fc fusion protein. PBL from this same leukophoresis were used to expand the dendritic cells (D.C.). These D.C. were cultured without pulsing with a tumor (Group 2), pulsed with the test tumor (Group 3), or pulsed with another human lung tumor (Group 4). Thirteen days later mice that had received PBL (xenotolerant or not) were injected I.P. with 2.5 x 10⁵ D.C. from groups 2-4, and Group 1 received no D.C. Two days later all mice were challenged with 2.5 x 10⁶ test tumor cells I.P. Six weeks later all the mice were sacrificed and examined for the presence of tumors. The aggregate weight of all tumor nodules in the peritoneal cavity was recorded. No tumor suppression was observed in mice that received PBL that were not xenotolerized (data not shown). In mice that received xenotolerant PBL and D.C. pulsed with test tumor 3 of the 5 mice had no tumor in the peritoneal cavity and two had tumor burdens of less than 1 gram . Mice that received xenotolerant PBL and a control tumor pulsed D.C. (Group 4) all had large tumor burdens. Mice from Groups 1 and 2 each had one mouse in the group that died with tumor prior to sacrifice and all the remaining mice in the groups had tumors (most over 1 gram) at the time of sacrifice.

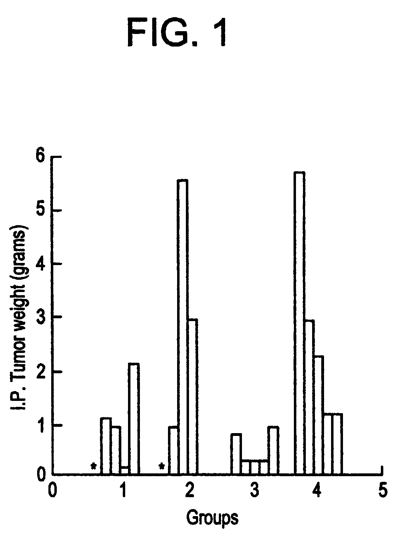

The results are shown in Figure 1. Group 1, no dendritic cell vaccination (5 mice, 1 died). Group 2, non-pulsed DC vaccinated (4 mice, 1 died). Group 3, test tumor pulsed DC vaccinated (5 mice, 3 tumor free). Group 4, control tumor pulsed DC vaccinated (5 mice). The significance of difference between groups was examined by Mann-Whitney test: P=0.0318 between groups 3 and 1; P=0.0179 between groups 3 and 2; P=0.0040 between groups 3 and 4; P=0.0019 between groups 3 and the combination of groups 1, 2 and 4.

### Example 2. Use of Xenotolerant Model to Evaluate huPBL Response to a Human Lung Tumor Xenograft.

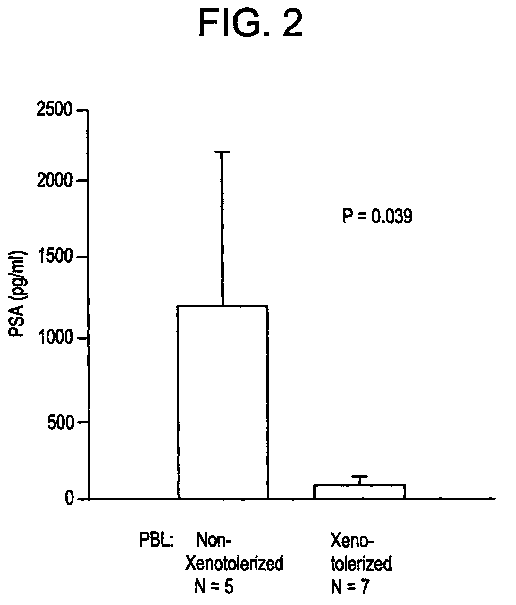

This example demonstrates the ability of xenotolerant (but not control) PBL to inhibit the growth of an allogeneic human lung tumor. This lung tumor was transfected with a mammalian expression vector containing the gene encoding human prostate specific antigen (PSA). These tumor xenografts produce and secrete PSA into the serum of tumor bearing SCID mice. SCID mice were engrafted intraperitoneally with 3 x 10⁷ xenotolerant or control (non-xenotolerant) PBL from a normal human donor. One week after huPBL inoculation mice were challenged with 2 x 10⁶ 2E9/PSA tumor cells injected intraperitoneally. This is a non-small cell lung tumor cell line that has been transfected with mammalian expression vector containing the gene for human prostate specific antigen (PSA). The PSA levels in the serum correlate with tumor growth. The results presented in Figure 2 were obtained 4 weeks after tumor challenge.

It was established that serum PSA levels can be monitored periodically and that increasing serum PSA levels correlate with the increasing size of the tumor xenograft. In this experiment the human PBL were from a normal donor and the tumor is allogeneic with respect to the huPBL. Note in Fig. 2 that the PSA level in the sera of the xenotolerized mice (i.e. tumor size) is suppressed compared to the PSA level in the mice that received control (non-xenotolerized) huPBL.

### Example 3. Use of Xenotolerant Model to Evaluate Epstein Barr Virus-Peptide Vaccination Strategy.

The following data are offered in further support of the notion that xenotolerance enhances the functional immune response of human PBL in the SCID mouse model. PBL from an EBV negative donor (5 x 10⁷) were inoculated I.P. into SCID mice with or without establishing xenotolerance with CD40Fc fusion protein. Mice were then divided into three groups. Group 1 received non-tolerant PBL and were vaccinated with a truncated form of the EBV peptide gp350/220. Group 2 mice received xenotolerant PBL but were not vaccinated. Group 3 mice received xenotolerant PBL and were vaccinated with the truncated EBV peptide. After vaccination all mice were challenged with wild type live EBV. All unvaccinated mice (Group 2) 8 of 8 developed lymphoma or died with lymphoma prior to sacrifice (Table 1). Vaccination of the group of mice receiving control (non-xenotolerant) PBL did not protect the mice from challenge with EBV (Group 1). Only mice in Group 3 i.e. xenotolerant PBL vaccinated with the EBV peptide showed significant protection from virus challenge with only 3 of 13 mice developing lymphomas.

**TABLE 1**

| Vaccination of Xenotolerant Human PBL in SCID Mice with EBV Peptide 350/220 Induces Virus Protective Immunity | | | | |
|---|---|---|---|---|
| Group | CD40-Fc | Vaccination | EBV Challenge | Lymphoma or Death/Total |
| 1 | - | EBV Peptide 350/220 | + | 13/15 |
| 2 | + | - | + | 8/8 |
| 3 | + | EBV Peptide 350/220 | + | 3/13 |

## Claims

1. A severe combined immunodeficient mammal comprising engrafted human lymphocytes, characterized in that said human lymphocytes have been rendered tolerant to mouse tissue antigens prior to said engrafting, said human lymphocytes having the ability to respond to non-mouse exogenous antigens from the time of said engrafting.

2. The mammal of claim 1, characterized in that the mammal is a mouse.

3. The immunodeficient mouse of claim 2 characterized in that the lymphocytes are human blood lymphocytes which are rendered tolerant to mouse tissue antigens prior to engrafting by blocking co-stimulatory molecules on said lymphocytes.

4. The immunodeficient mouse of claim 3 characterized in that the co-stimulatory blocking is achieved by the addition of human CD40Fc fusion protein to the lymphocytes to bind the CD40 ligand on the lymphocytes and then exposing the lymphocytes, with their CD40 sites blocked, to mouse tissue antigens.

5. A method for monitoring human blood lymphocytic immune response characterized in that the mammal of claim 1 with an antigen normally foreign to both human blood lymphocytes and to the mammal.

6. The method of claim 5 characterized in that the mammal is a mouse

7. The method of claim 6 characterized in that the antigen is found within a vaccine.

8. The method of claim 6 characterized in that the antigen is found within a tumor.

9. The method of claim 6 characterized in that the antigen is found within a virus.

10. A method for monitoring human lymphocytic immune response by challenging a mammal with an antigen normally foreign to said mammal and to human lymphocytes, said mammal being severely immune deficient characterized in that the mammal contains engrafted human lymphocytes which are tolerant of antigens from the mammal but otherwise have a normal human immune response to exogenus antigens.
